# EUROPEAN PATENT APPLICATION

(11) **EP 4 471 145 A1**
(43) Date of publication of application: **04.12.2024**
(21) Application number: 23746357.5
(22) Date of filing: 27.01.2023
(51) Int. Cl.: C12N 15/113, A61K 31/7088, A61P 3/06

(54) **SMALL RNA AND USE THEREOF IN TREATMENT OF HYPERLIPIDEMIA**

(30) Priority: 28.01.2022 CN 202210106396
(71) Applicant: Institute of Basic Medical Sciences, Chinese Academy of Medical Sciences, Beijing 100005 (CN)
(72) Inventor: JIANG, Chengyu, Beijing 100005 (CN); TANG, Kegong, Beijing 100005 (CN); CHEN, Mingrui, Beijing 100005 (CN)
(74) Representative: Cabinet Beau de Loménie
(86) International application number: PCT/CN2023/073495
(87) International publication number: WO 2023/143479

(57) **Abstract**

Provided are small RNA and a use thereof in the treatment of hyperlipidemia. Specifically, provided are an isolated nucleic acid molecule as shown in any one of SEQ ID NO: 1 to SEQ ID NO: 75, a vector comprising same, a host cell, a pharmaceutical composition, and a use of the nucleic acid molecule in the preparation of a drug for treating hyperlipidemia.

## Description

The present application claims the priority of the Chinese patent application CN202210106396.2 filed on January 28, 2022, and the full text of the above Chinese patent application is cited in the present application.

### FIELD OF THE INVENTION

The present invention generally relates to the field of nucleic acid therapy. More specifically, the present invention relates to new small RNA and use thereof in treatment of hyperlipidemia.

### BACKGROUND OF THE INVENTION

Hypercholesterolemia is a common dyslipidemia characterized by elevated levels of low density lipoprotein C (LDL-C). It is well known to be directly associated with an increased risk of atherosclerotic cardiovascular disease. LDL-C transports cholesterol through the bloodstream and deposits it on the artery walls, which increases atherosclerosis, thereby leading to plaque formation. The main goal of hypercholesterolemia treatment is to reduce blood LDL-C levels, thereby reducing plaque formation and thus reducing the morbidity and mortality of cardiovascular diseases.

The traditional treatment is the use of statins. These drugs increase the expression of LDL receptors by inhibiting HMG-CoA reductase, thereby increasing the clearance of LDL particles by the liver. However, some patients show intolerance to statins, and statins can cause side effects such as myalgia or hepatotoxicity.

Proprotein convertase subtilisin/kexin type 9 (PCSK9) is a serine protease expressed by the liver and secreted into the bloodstream. The binding of PCSK9 to the LDL receptor makes the LDL receptor more susceptible to cleavage by cysteine cathepsin and intracellular degradation. Therefore, the expression of PCSK9 and its release into the circulation increase plasma LDL concentrations. Inclisiran is a fully chemically stable double-stranded RNA that targets the 3' UTR of PCSK9 mRNA. Inclisiran is conjugated to N-acetylgalactosamine carbohydrates, making it specifically uptake by hepatocytes. It is a small interfering ribonucleic acid (siRNA) molecule for treating hyperlipidemia. Inclisiran has now been shown to reduce LDL-cholesterol levels in patients for 6 months by a single subcutaneous injection, and is therefore used as a positive drug control in the present invention.

However, injectable drugs always give patients a poor experience compared with oral drugs. In previous research, the research team of the inventor has discovered that a variety of compounds extracted from medicinal plants are capable of promoting the delivery or synthesis of nucleic acids, and nucleic acids such as sRNA can be orally delivered to the target site in the body of the subject in need thereof using the extracted compounds or a variety of combinations. In the present invention, a natural medicinal RNA that is completely complementary and matched with PCSK9 is screened from a medicinal plant decoction sRNA library, which has a significant effect in treating hyperlipidemia, and can also exert hypoglycemic effect by targeting GCGR.

### SUMMARY OF THE INVENTION

The present application is partially based on the inventor's discovery of a series of small RNAs of medicinal plants. The small RNA isolated by the inventors from medicinal plants can play a role in lowering blood lipids in a high-fat mouse model.

The inventors use an obese mouse model, i.e., the ob/ob mouse model, to screen small RNAs with hypolipidemic function isolated from the traditional Chinese medicine chicory.

The inventors use a beagle dog model and a hyperlipidemic cynomolgus monkey model to verify the function of the small RNAs isolated from the traditional Chinese medicine chicory to improve blood sugar and blood lipids in large animals.

Compared with Inclisiran, the small RNAs provided by the present invention have the effect of significantly improving glucose tolerance, enhancing sensitivity to insulin, reducing serum triglyceride and low density lipoprotein levels, increasing high density lipoprotein level, reducing liver lipid accumulation, as well as reducing liver tissue inflammatory factor level, and improving liver function.

Based on the above discoveries, in a first aspect, the present invention provides an isolated nucleic acid molecule comprising or consisting of the following sequences:
A) a nucleotide sequence as shown in any one of SEQ ID NO: 1 to SEQ ID NO: 75;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 75;
C) a sequence having at least 10, preferably at least 9, at least 8, at least 7, at least 6, at least 5, at least 4, at least 3, at least 2, at least 1 nucleotide substitutions, deletions, or additions compared with a nucleotide sequence as shown in any one of SEQ ID NO: 1 to SEQ ID NO: 75,
D) a sequence capable of hybridizing to a nucleotide sequence as shown in any one of SEQ ID NO: 1 to SEQ ID NO: 75 under stringent conditions, or
E) a nucleotide sequence targeting the PCSK9 or GCGR gene, preferably a nucleotide sequence capable of binding complementarily to a nucleotide sequence as shown in SEQ ID NO: 76 or 77.

In a preferred embodiment, the isolated nucleic acid molecule according to the present invention exerts a hypolipidemic effect by targeting PCSK9, preferably by binding complementarily to the CDR, 3' UTR or 5' UTR of PCSK9 to reduce the expression of PCSK9, and preferably by binding complementarily to the CDS region of PCSK9 as shown in SEQ ID NO: 76 to reduce the expression of PCSK9.

In preferred embodiments, the isolated nucleic acid molecule according to the present invention is an RNA molecule or a DNA molecule, preferably a small RNA molecule, preferably a small RNA molecule with a length of 18-24 nucleotides, preferably a small RNA molecule with a length of 19, 20, 21, 22, 23, or 24 nucleotides.

In one embodiment, the present invention provides an isolated small RNA molecule targeting the PCSK9 gene and comprising or consisting of the following sequence:
A) a nucleotide sequence as shown in any one of SEQ ID NO: 1 to SEQ ID NO: 75;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 75;
C) a sequence having up to 10 nucleotide substitutions, deletions, or additions compared with a nucleotide sequence as shown in any one of SEQ ID NO: 1 to SEQ ID NO: 75, preferably a sequence having 9, 8, 7, 6, 5, 4, 3, 2, 1 nucleotide substitutions, deletions, or additions,
D) a sequence capable of hybridizing to a nucleotide sequence as shown in any one of SEQ ID NO: 1 to SEQ ID NO: 75 under stringent conditions, or
E) a nucleotide sequence capable of binding complementarily to a nucleotide sequence as shown in SEQ ID NO: 76 or 77.

In particular embodiments, the small RNA molecule provided by the present invention binds complementarily to the CDR, 3' UTR or 5' UTR of PCSK9.

In particular embodiments, the present invention provides a small RNA molecule targeting the nucleotide sequence as shown in SEQ ID NO: 76 or 77.

In particular embodiments, the present invention provides an isolated small RNA molecule comprising or consisting of the following sequence:
A) a nucleotide sequence as shown in any one of SEQ ID NO: 1 to SEQ ID NO: 7;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 7, and binding complementarily to the nucleotide sequence as shown in SEQ ID NO: 76 or 77;
C) a sequence having up to 10 nucleotide substitutions, deletions, or additions compared with a nucleotide sequence as shown in any one of SEQ ID NO: 1 to SEQ ID NO: 7, preferably a sequence having 9, 8, 7, 6, 5, 4, 3, 2, 1 nucleotide substitutions, deletions, or additions, and binding complementarily to the nucleotide sequence as shown in SEQ ID NO: 76 or 77; or
D) a sequence capable of hybridizing to a nucleotide sequence as shown in any one of SEQ ID NO: 1 to SEQ ID NO: 7 under stringent conditions.

In the embodiments of the present invention, the isolated small RNA molecule of the present invention exerts a hypolipidemic, hypoglycemic and/or hypotensive function by targeting PCSK9 CDS and/or GCGR CDS.

In the embodiments of the present invention, the small RNA molecule of the present invention may be in the form of single-stranded or double-stranded.

The double-stranded small RNA molecule of the present invention comprises a sense strand and an antisense strand, wherein the sense strand comprises or consists of the following sequence:
A) a nucleotide sequence as shown in any one of SEQ ID NO: 1 to SEQ ID NO: 75;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 75;
C) a nucleotide sequence having up to 10 nucleotide substitutions, deletions, or additions compared with a nucleotide sequence as shown in any one of SEQ ID NO: 1 to SEQ ID NO: 75, preferably a nucleotide sequence having 9, 8, 7, 6, 5, 4, 3, 2, 1 nucleotide substitutions, deletions, or additions compared with a nucleotide sequence as shown in any one of SEQ ID NO: 1 to SEQ ID NO: 75,
D) a nucleotide sequence capable of hybridizing to a nucleotide sequence as shown in any one of SEQ ID NO: 1 to SEQ ID NO: 75 under stringent conditions, or
E) a nucleotide sequence capable of binding complementarily to a nucleotide sequence as shown in SEQ ID NO: 76 or 77; and
the antisense strand comprises a sequence complementary to the sense strand.

In another aspect, the present invention provides a precursor miRNA, which may be processed within the host into the isolated small RNA molecule according to the present invention.

In another aspect, the present invention provides a polynucleotide, which may be transcribed by the host to form the precursor miRNA according to the present invention.

In another aspect, the present invention provides an expression vector comprising the nucleic acid molecule, the isolated small RNA molecule, the precursor miRNA, and/or the polynucleotide of the present invention.

In another aspect, the present invention provides a host cell transfected with the expression vector according to the present invention.

In another aspect, the present invention provides a pharmaceutical composition comprising a therapeutically effective amount of the nucleic acid molecule, the small RNA molecule, the precursor miRNA, the polynucleotide, the expression vector or the host cell according to the present invention, as well as one or more pharmaceutically acceptable adjuvants, excipients and/or stabilizers. Preferably, the pharmaceutical composition according to the present invention may be used for administration via oral, intramuscular, intravenous, subcutaneous, percutaneous, intraarterial, intraperitoneal, intrapulmonary, intracerebrospinal, intraarticular, intrasynovial, intrathecal, intraventrical, and/or inhalation routes; preferably, the composition according to the present invention is administered orally.

Preferably, in the pharmaceutical composition of the present invention, the content of the nucleic acid molecule, the small RNA molecule, the precursor miRNA, the polynucleotide, the expression vector or the host cell is 0.1-1000 µM, preferably 3.0 µM-300 µM, preferably 0.3 µM, 0.6 µM, 0.9 µM, 1.0 µM, 3.0 µM, 6.0 µM, 9.0 µM, 10.0 µM, 13.0 µM, 16.0 µM, 19.0 µM, 20.0 µM, 23.0 µM, 26.0 µM, 29.0 µM, 30.0 µM, 33.0 µM, 36.0 µM, 39.0 µM, 40.0 µM, 43.0 µM, 46.0 µM, 49.0 µM, 50.0 µM, 53.0 µM, 56.0 µM, 59.0 µM, 60.0 µM, 63.0 µM, 66.0 µM, 69.0 µM, 70.0 µM, 73.0 µM, 76.0 µM, 79.0 µM, 80.0 µM, 83.0 µM, 86.0 µM, 89.0 µM, 90.0 µM, 100 µM, 130 µM, 160 µM, 190 µM, 200 µM, 250 µM, 300 µM, 350 µM, 400 µM, 450 µM, 500 µM, 550 µM, 600 µM, 650 µM, 700 µM, 750 µM, 800 µM, 850 µM, 900 µM, 950 µM, 1000 µM, or any range between these point values.

In another aspect, the present invention provides use of the isolated nucleic acid molecule, the small RNA molecule, the precursor miRNA, the polynucleotide, the expression vector, the host cell or the pharmaceutical composition according to the present invention in the preparation of a medicament for treating hyperlipidemia in a subject; preferably, the isolated nucleic acid molecule, the vector, the cell or the pharmaceutical composition according to the present invention may be used to improve serum high levels of low density lipoprotein and triglyceride, and simultaneously reduce blood glucose, and/or protect the subject from liver injury caused by hyperlipidemia.

Preferably, the isolated nucleic acid molecule, the small RNA molecule, the precursor miRNA, the polynucleotide, the expression vector, the host cell or the pharmaceutical composition according to the present invention may be used in combination with one or more, preferably one to three other drugs, for treating hyperlipidemia, said other drugs are selected from the group consisting of statins, cholesterol transport inhibitors, bile acid chelators, phenoxyaromatic acids, nicotinic acids, CETP inhibitors, MTP inhibitors and other PCSK9 inhibitor drugs.

The isolated nucleic acid molecule, the small RNA molecule, the precursor miRNA, the polynucleotide, the expression vector, the host cell or the pharmaceutical composition provided by the present invention may be used for treating and/or preventing a disease associated with elevated blood lipids, including but not limited to, the treatment and/or prevention of atherosclerosis, coronary heart disease, myocardial infarction, cerebral hemorrhage, and cerebral thrombosis.

In another aspect, the present invention provides use of the isolated nucleic acid molecule, the small RNA molecule, the precursor miRNA, the polynucleotide, the expression vector, the host cell or the pharmaceutical composition according to the present invention in the preparation of a medicament for treating hypertension in a subject.

In another aspect, the present invention provides use of the isolated nucleic acid molecule, the small RNA molecule, the precursor miRNA, the polynucleotide, the expression vector, the host cell or the pharmaceutical composition according to the present invention in the preparation of a medicament for treating diabetes in a subject; preferably, the isolated nucleic acid molecule, the vector, the cell or the pharmaceutical composition according to the present invention may be used to improve glucose tolerance and enhance sensitivity to insulin.

Preferably, the isolated nucleic acid molecule, the small RNA molecule, the precursor miRNA, the polynucleotide, the expression vector, the host cell or the pharmaceutical composition according to the present invention may be used in combination with one or more, preferably one to three other drugs, for treating diabetes, said other drugs are selected from the group consisting of insulin and analogues thereof, sulfonylurea secretagogues, metformins, α-glucosidase inhibitors, thiazolidinedione derivative sensitizers, phenylanisic acid derivative secretagogues, GLP-1 receptor agonists and DPP-4 enzyme inhibitors.

In another aspect, the present invention provides a method for treating hyperlipidemia in a subject in need, including administering to the subject a therapeutic effective amount of the isolated nucleic acid molecule, the small RNA molecule, the precursor miRNA, the polynucleotide, the expression vector, the host cell or the pharmaceutical composition according to the present invention; preferably, as used herein, the term "subject" is a vertebrate, more preferably a mammal, even more preferably a domestic animal or a companion animal, such as chicken, goose, duck, goat, sheep, cattle, pig, horse, dog, cat, hamster, rat, mouse, hamster, or guinea pig. Most preferably, the subject is a human.

In another aspect, the present invention provides a method for treating hypertension in a subject in need, including administering to the subject a therapeutic effective amount of the isolated nucleic acid molecule, the small RNA molecule, the precursor miRNA, the polynucleotide, the expression vector, the host cell or the pharmaceutical composition according to the present invention.

In another aspect, the present invention provides a method for treating diabetes in a subject in need, including administering to the subject a therapeutic effective amount of the isolated nucleic acid molecule, the small RNA molecule, the precursor miRNA, the polynucleotide, the expression vector, the host cell or the pharmaceutical composition according to the present invention.

In another aspect, the present invention provides a method for treating and/or preventing a disease associated with elevated blood lipids in a subject in need, including administering to the subject a therapeutic effective amount of the isolated nucleic acid molecule, the isolated small RNA molecule, the precursor miRNA, the polynucleotide, the expression vector, the host cell or the pharmaceutical composition according to the present invention, the disease associated with elevated blood lipids is selected from the group consisting of atherosclerosis, coronary heart disease, myocardial infarction, cerebral hemorrhage, and cerebral thrombosis.

In another aspect, provided is a method for treating a disease associated with abnormal gene expression, including administering to the subject a therapeutic effective amount of the nucleic acid molecule, the small RNA molecule, the precursor miRNA, the polynucleotide, the expression vector, the host cell or the pharmaceutical composition according to the present invention.

In another aspect, the present invention provides a method for inhibiting the expression and/or activity of a gene, including administering to a subject a therapeutically effective amount of the nucleic acid molecule, the small RNA molecule, the precursor miRNA, the polynucleotide, the expression vector, the host cell or the pharmaceutical composition according to the present invention.

Unless stated otherwise, the terms used herein have the meanings generally understood by those skilled in the art.

### Terms

Generally, siRNA, miRNA and other non-coding small RNAs are indiscriminately referred to as oligonucleotides or small RNAs (sRNAs). As used herein, "small RNAs" are a large group of small, non-coding RNAs encoded within animal and plant genomes, with a length of about 18-24 nucleotides. Studies have shown that small RNAs are involved in a wide variety of regulatory pathways, including development, viral defense, hematopoietic processes, organ formation, cell proliferation and apoptosis, fat metabolism, etc.

As used herein, a small RNA (sRNA) can be a single-stranded or double-stranded RNA, including but not limited to siRNA and miRNA, which may be a natural or synthetic RNA.

As used herein, the term "nucleic acid" includes "polynucleotide", "oligonucleotide" and "nucleic acid molecule", and generally refers to DNA or RNA polymers, which may be single-stranded or double-stranded, synthetic or obtained from natural sources (e.g., isolated and/or purified); it may comprise natural, unnatural, or altered nucleotides. In some embodiments, a nucleic acid does not comprise any insertion, deletion, inversion, and/or substitution. However, as discussed herein, in some cases it may be appropriate for a nucleic acid to comprise one or more insertions, deletions, inversions, and/or substitutions.

As used herein, the term "hybridizing under stringent conditions" means that a nucleotide sequence specifically hybridizes to a target sequence (e.g., a sequence as shown in SEQ ID NO: 1) in an amount that is detectably stronger than non-specific hybridization. Stringent conditions can include, for example, low salt and/or high temperature conditions, such as those provided by about 0.02 M to 0.1 M of NaCl or equivalent substances at a temperature of about 50°C to 70°C.

As used herein, "sequence identity" refers to the sequence similarity between two polynucleotide sequences. When the positions in the two sequences aligned are occupied by the same base, for example if each position of two DNA molecules is occupied by adenine, then the molecules are identical at that position. The identity percentage between two sequences is a function of the number of matching or homologous positions shared by the two sequences divided by the number of positions compared × 100.

As used herein, the term "vector" refers to a recombinant expression vector that incorporates the nucleic acid described herein. The recombinant expression vector may be any suitable recombinant expression vector and may be used to transform or transfect any suitable host cell, including but not limited to plant expression vector, animal expression vector, viral vector such as retroviral vector or lentiviral vector. These vectors are well known to those skilled in the art and are commercially available.

As used herein, the term "host cell" refers to any type of cell that can be transfected with a recombinant expression vector according to the present invention. The host cell can be an eukaryotic cell, such as plant, animal, fungus, or alga, or it can be a prokaryotic cell, such as bacterium or protozoan.

A variety of transfection techniques are well known in the art, including but not limited to calcium phosphate co-precipitation, direct microinjection into cultured cells, electroporation, liposome-mediated gene transfer, lipid-mediated transduction, and nucleic acid delivery using high-speed microprojectiles.

As used herein, "treating" or "treatment" includes treating a disease state in a mammal, particularly in a human, and includes: (a) suppressing the disease state, i.e., preventing its progression; and/or (b) alleviating the disease state, i.e., causing the disease state to regress.

As used herein, the term "subject" refers to any human or non-human organism that may potentially benefit from treatment with the nucleic acid molecule of the present invention or the vector, cell, or composition comprising the same. Exemplary subjects include subjects with diabetes. Preferably, the term "subject" as used herein is a vertebrate, preferably a mammal, even more preferably a domestic animal or a companion animal, such as chicken, goose, duck, goat, sheep, cattle, pig, horse, dog, cat, hamster, rat, mouse, hamster, or guinea pig. Most preferably, the subject is a human.

As used herein, the term "therapeutic effective amount" is intended to include the amount of the nucleic acid molecule of the present invention or the vector, cell or composition comprising the same, which would benefit a subject when the nucleic acid molecule of the present invention or the vector, cell or composition comprising the same is administered alone or in combination.

As used herein, the term "complementary binding" or "binding complementarily" means that the two single strands form a detectable double strand by base pairing with each other. However, as long as a stable double strand can be formed, a certain percent of mismatches between the two single strands is allowed. For example, in some embodiments, the double strand has about 1%, about 2%, about 3%, about 4%, about 5%, about 6%, about 7%, about 8%, about 9%, about 10%, about 15%, about 20%, about 25%, about 30%, about 35%, about 40%, about 45%, or about 50% of mismatches.

The specific embodiments below are provided in the present specification in order to illustrate the present invention in more details, and are illustrated with reference to the attached drawings, but the solutions of the present disclosure is not limited thereto. Those skilled in the art may, on the basis of the general knowledge in the art, make appropriate alterations to the method, use and small RNA of the present invention, which should fall within the scope of the present invention as long as they can realize the functions described in the present invention.

As used herein, the terms "high blood lipids", "hyperlipidemia", and "elevated blood lipids" are used interchangeably and refer to a disease of systemic metabolic abnormalities, in which plasma cholesterol, triglycerides, and low density lipoprotein levels are elevated while high density lipoprotein is abnormally low due to various factors.

Hyperlipidemia can be classified into primary and secondary hyperlipidemia. Primary hyperlipidemia is hyperlipidemia that occurs in the absence of any other previous disease, usually due to genetic factors; secondary hyperlipidemia is hyperlipidemia caused by various factors, such as diabetes, hypothyroidism, nephrotic syndrome, kidney transplantation, biliary obstruction, etc. Hyperlipidemia can be caused by poor dietary habits, such as overeating and overdrinking, alcoholism, dietary bias, irregular diet, etc. Hyperlipidemia can also be caused by long-term use of certain drugs, such as contraceptives, hormone drugs, etc. Hyperlipidemia caused by mental factors is due to long-term mental stress, which leads to endocrine and metabolic disorders, thereby developing hyperlipidemia.

### DESCRIPTION OF THE DRAWINGS

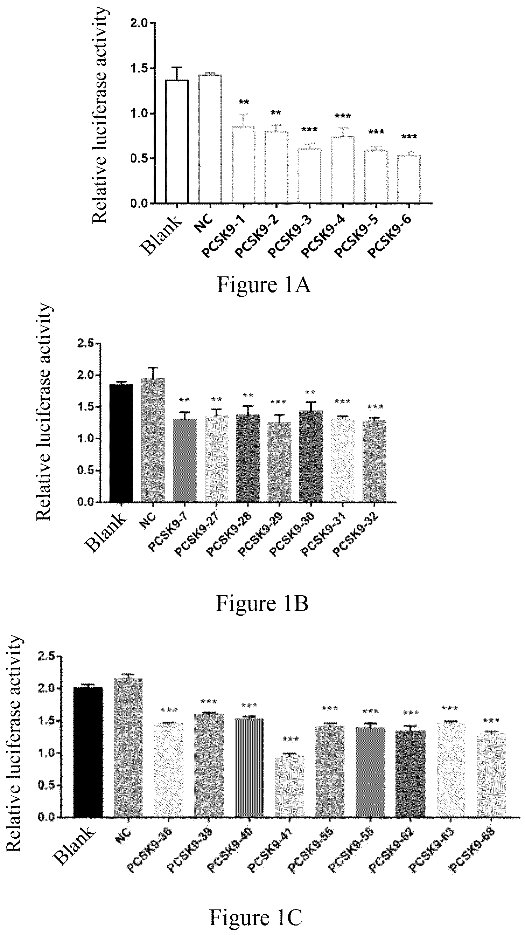

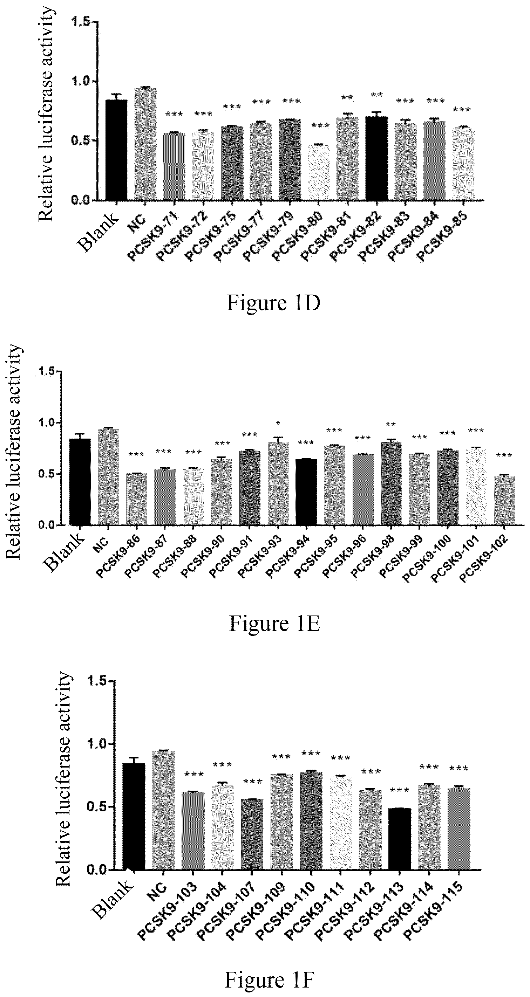

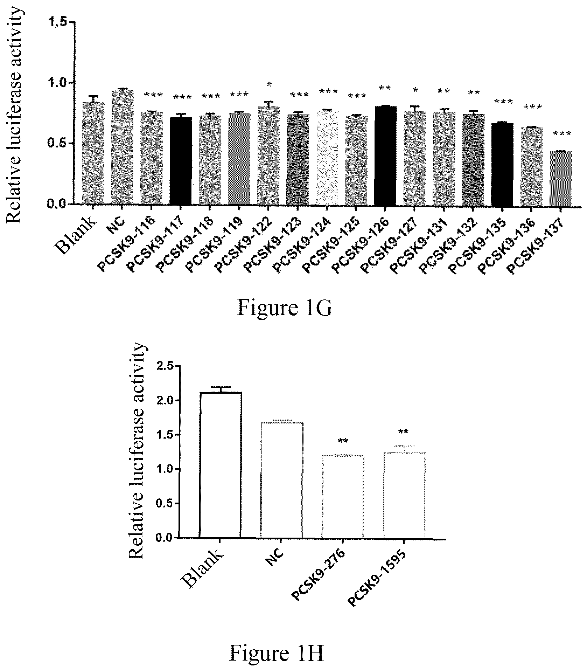

Figure 1A to Figure 1H show the targeting effect of the small RNA of the present invention on proprotein convertase subtilisin/kexin type 9 (PCSK9) as detected by a dual luciferase reporter detection system. Blank indicates no transfection of small RNA, and NC is the irrelative control sequence group. The horizontal axis in the figure represents the name of the small RNA bound to PCSK9, for example, PCSK9-7 represents the small RNA PCSK9-sRNA-7 of the present invention, and so forth. PCSK9-7 is also named as Juju-JzJt-1;

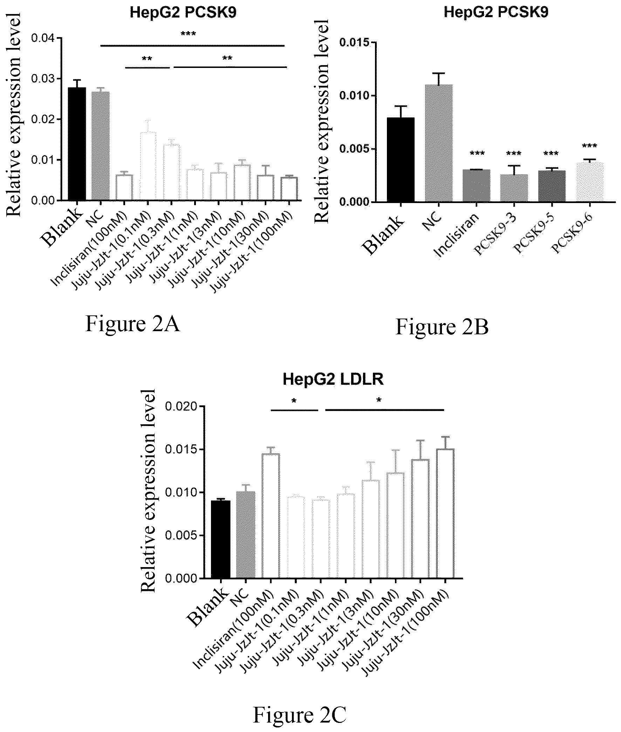

Figure 2A to Figure 2C show the effect of the small RNAs Juju-JzJt-1, PCSK9-3, PCSK9-5, and PCSK9-6 of the present invention on the mRNA levels of the target genes *PCSK9* and *LDLR* on HepG2 (human hepatic cancer cells) as detected by reverse transcription-real-time PCR compared with Inclisiran. Blank represents no transfection with small RNAs, NC is the irrelative control sequence group; Inclisiran represents the positive control group; Juju-JzJt-1 (0.1 nM, 0.3 nM, 1 nM, 3 nM, 10 nM, 30 nM, 100 nM) represents the experimental groups of different doses; PCSK9-3, PCSK9-5, PCSK9-6 represent the experimental groups of a dose of 100 nM;

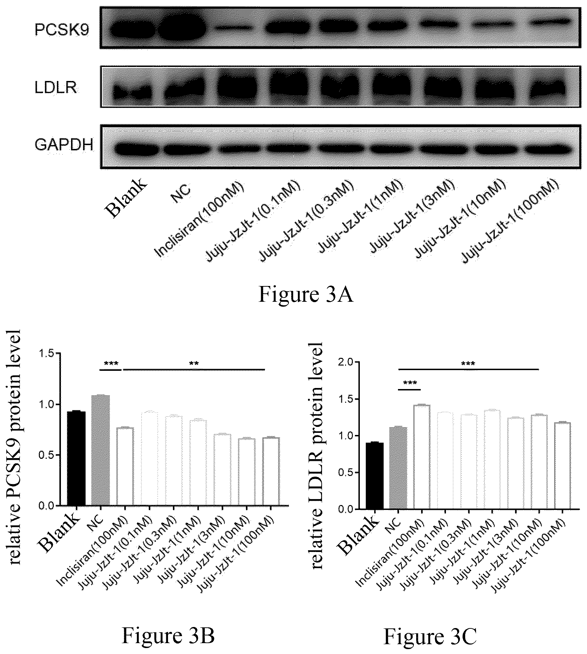

Figure 3A, Figure 3B and Figure 3C show the effect of the small RNA Juju-JzJt-1 of the present invention on the protein levels of the target genes *PCSK9* and *LDLR* on HepG2 cells as detected by western blot compared with Inclisiran. Blank represents no transfection with small RNAs, NC is the irrelative control sequence group; Inclisiran represents the positive control group; Juju-JzJt-1 (0.1 nM, 0.3 nM, 1 nM, 3 nM, 10 nM, 100 nM,) represents the experimental groups of different doses;

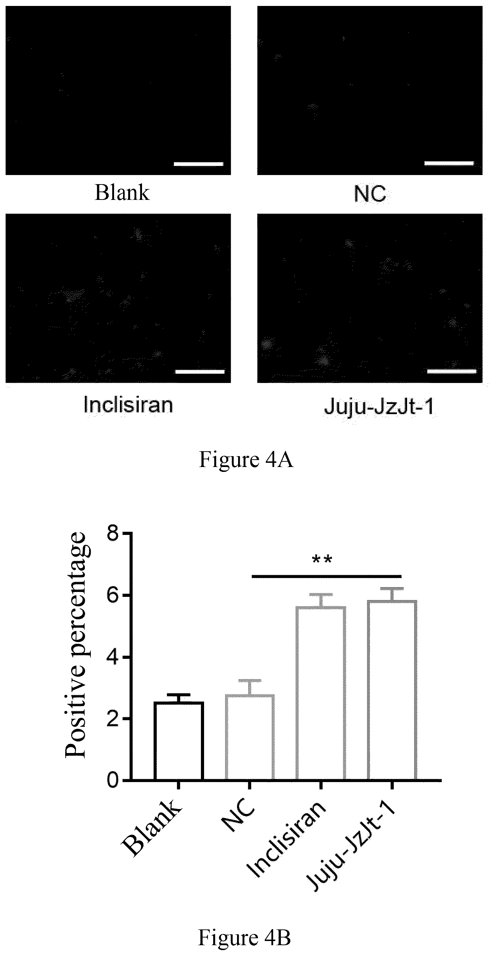

Figure 4A and Figure 4B show the effect of the small RNA Juju-JzJt-1 of the present invention on the uptake of human-derived low density lipoprotein labeled with red fluorescence on HepG2 cells compared with Inclisiran. Blank represents no transfection with small RNAs, NC is the irrelative control sequence group; Inclisiran represents the positive control group; Juju-JzJt-1 represents the experimental group;

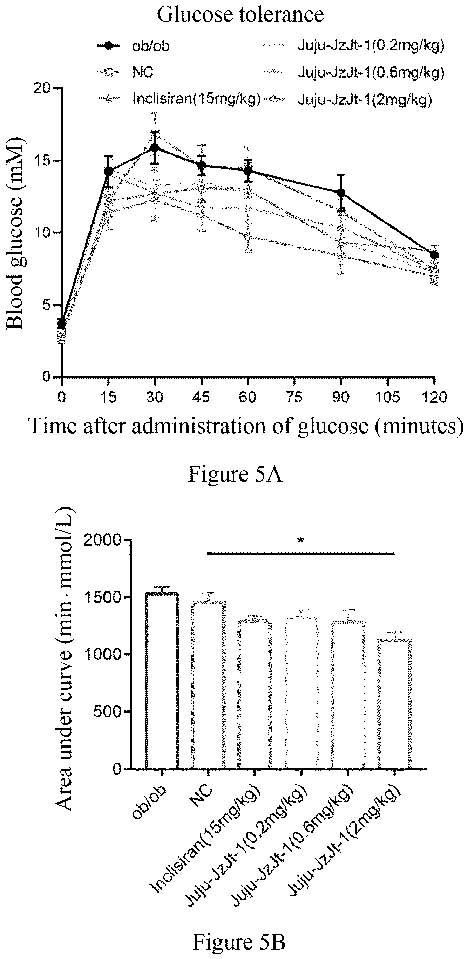

Figure 5A to Figure 5D show the effect of the small RNAs Juju-JzJt-1 and PCSK9-3 of the present invention on oral glucose tolerance in the high-fat model ob/ob mice compared with Inclisiran, wherein ob/ob represents the model group; NC represents the negative control group; Inclisiran (15 mg/kg) represents the positive control group; Juju-JzJt-1 (0.2 mg/kg, 0.6 mg/kg, 2 mg/kg) represents the experimental groups of different doses; PCSK9-3 represents the experimental group of a dose of 2 mg/kg;

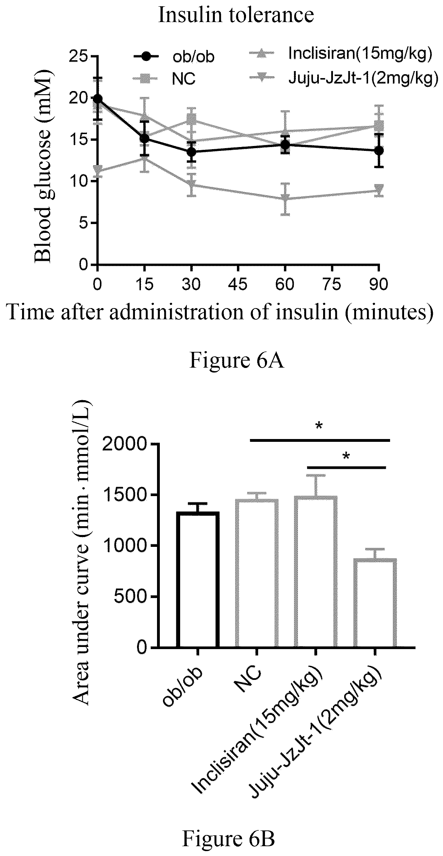

Figure 6A and Figure 6B show the effect of the small RNA Juju-JzJt-1 of the present invention on insulin tolerance in the high-fat model ob/ob mice compared with Inclisiran; wherein ob/ob represents the model group; NC represents the negative control group; Inclisiran (15 mg/kg) represents the positive control group; Juju-JzJt-1 (2 mg/kg) represents the experimental group;

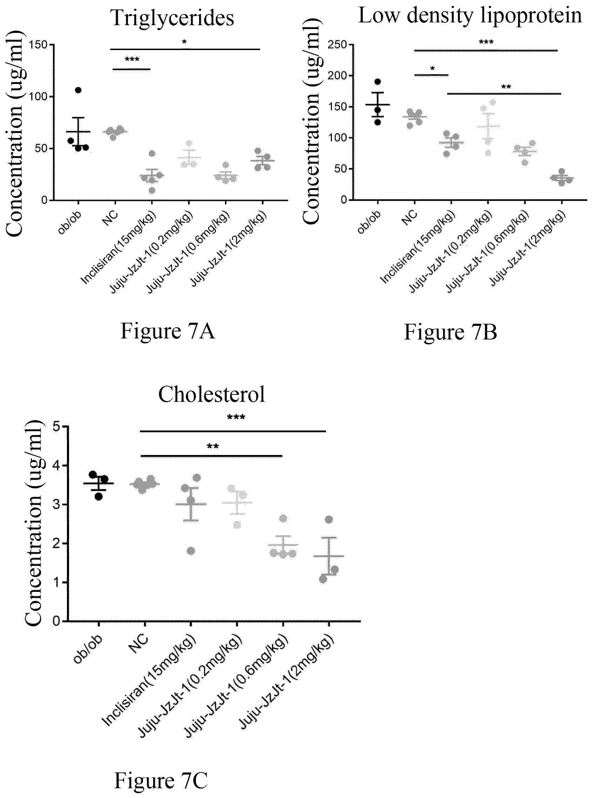

Figure 7A, Figure 7B and Figure 7C show the effect of the small RNA Juju-JzJt-1 of the present invention on serum triglycerides, low density lipoprotein and cholesterol in the high-fat model ob/ob mice compared with Inclisiran, wherein ob/ob represents the model group; NC represents the negative control group; Inclisiran (15 mg/kg) represents the positive control group; Juju-JzJt-1 (0.2 mg/kg, 0.6 mg/kg, 2 mg/kg) represents the experimental groups of different doses;

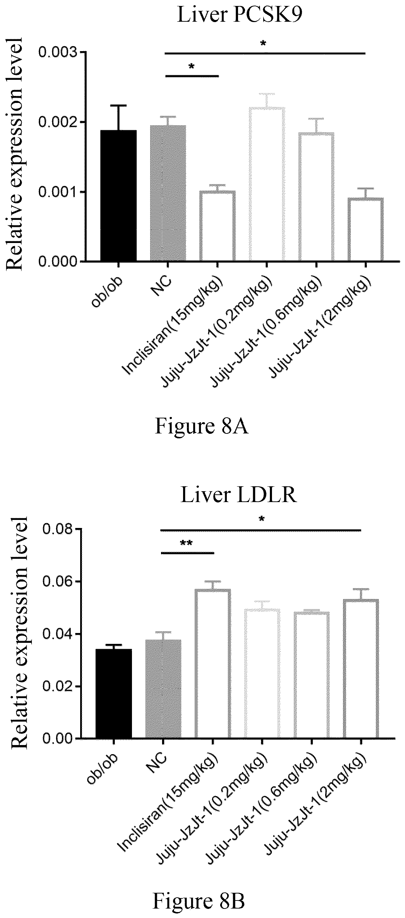

Figure 8A and Figure 8B show the effect of the small RNA Juju-JzJt-1 of the present invention on the messenger RNA levels of the target genes *PCSK9* and *LDLR* as detected by reverse transcription-real-time PCR in mouse liver compared with Inclisiran, wherein ob/ob represents the model group; NC represents the negative control group; Inclisiran (15 mg/kg) represents the positive control group; Juju-JzJt-1 (2 mg/kg) represents the experimental group;

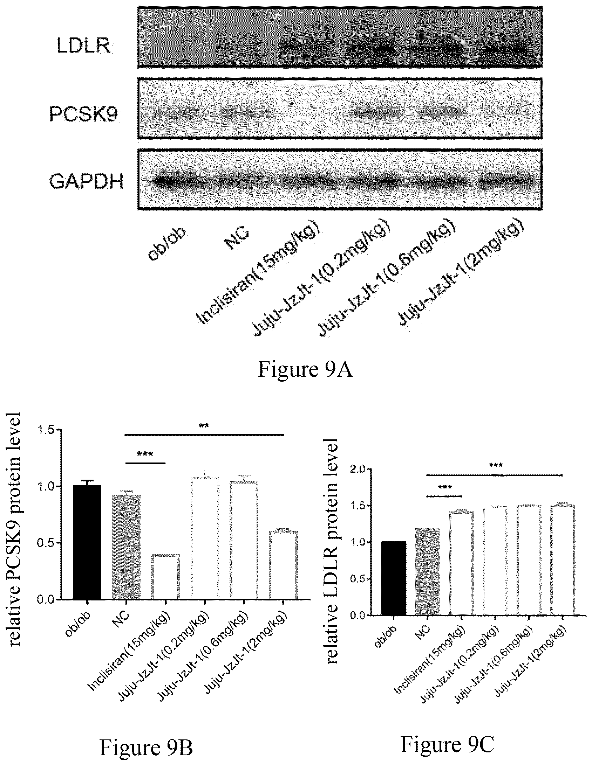

Figure 9A, Figure 9B and Figure 9C show the effect of the small RNA Juju-JzJt-1 of the present invention on the protein levels of the target genes *PCSK9* and *LDLR* on HepG2 cells as detected by western blot compared with Inclisiran, wherein ob/ob represents the model group; NC represents the negative control group; Inclisiran (15 mg/kg) represents the positive control group; Juju-JzJt-1 (2 mg/kg) represents the experimental group;

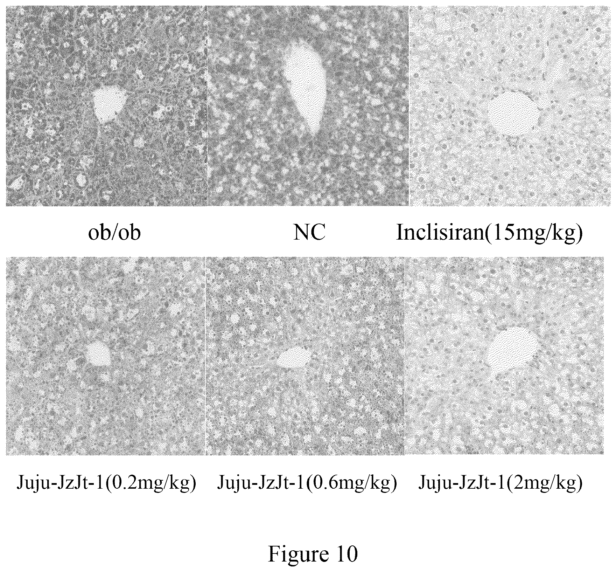

Figure 10 shows the effect of the small RNA Juju-JzJt-1 of the present invention on the oil red O staining of the high-fat model ob/ob mouse liver, wherein ob/ob represents the model group; NC represents the negative control group; Inclisiran (15 mg/kg) represents the positive control group; Juju-JzJt-1 (2 mg/kg) represents the experimental group;

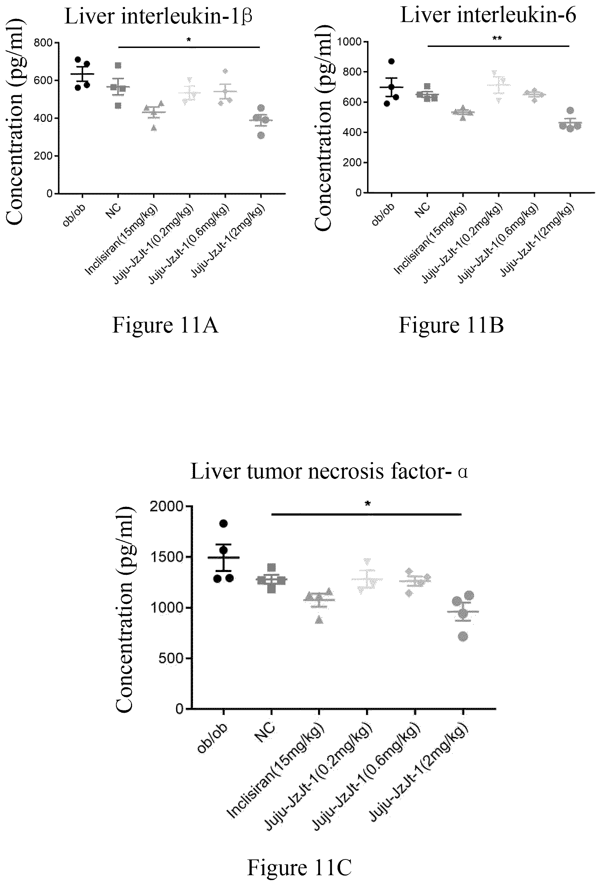

Figure 11A, Figure 11B and Figure 11C show the effect of the small RNA Juju-JzJt-1 of the present invention on the inflammatory cytokines interleukin-1β, interleukin-6, tumor necrosis factor-α in the high-fat model ob/ob mouse liver tissues compared with Inclisiran, wherein ob/ob represents the model group; NC represents the negative control group; Inclisiran (15 mg/kg) represents the positive control group; Juju-JzJt-1 (0.2 mg/kg, 0.6 mg/kg, 2 mg/kg) represents the experimental groups of ob/ob + different doses of Juju-JzJt-1;

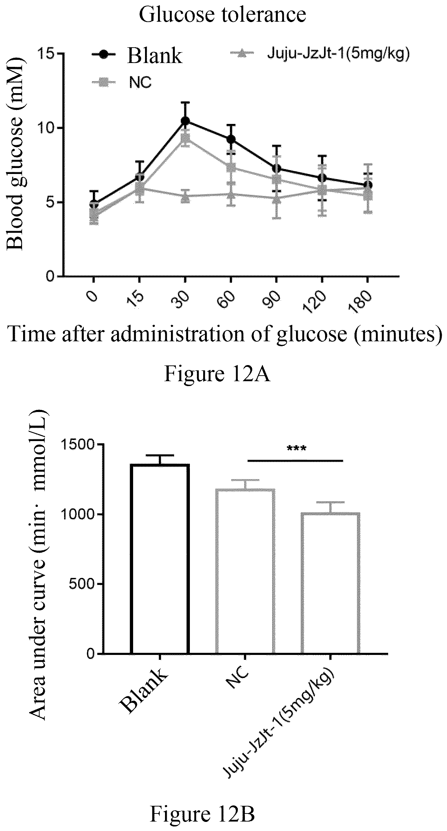

Figure 12A and Figure 12B show the effect of the small RNA Juju-JzJt-1 of the present invention on oral glucose tolerance in beagle dogs; wherein blank represents no treatment; NC represents the negative control group; Juju-JzJt-1 (5 mg/kg) represents the experimental group;

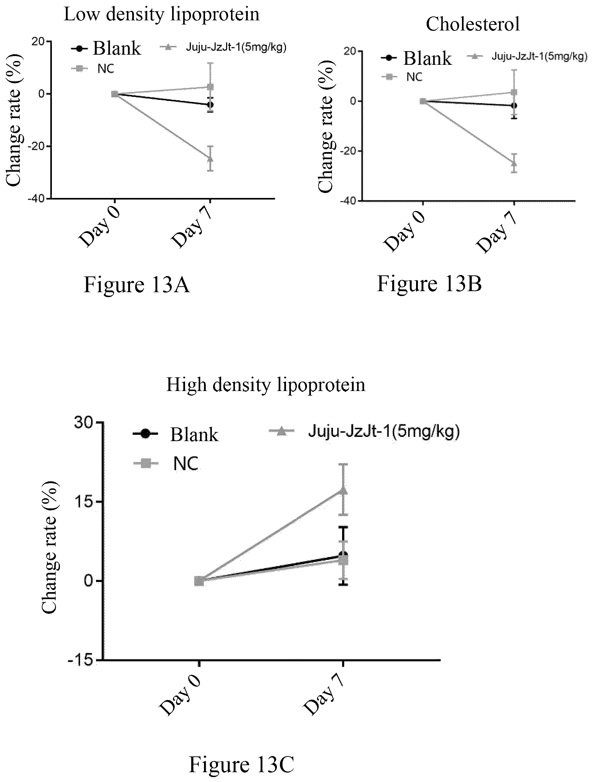

Figure 13A, Figure 13B and Figure 13C show the effect of the small RNA Juju-JzJt-1 of the present invention on serum low density lipoprotein, cholesterol, and high density lipoprotein in beagle dogs; wherein blank represents no treatment; NC represents the negative control group; Juju-JzJt-1 (5 mg/kg) represents the experimental group;

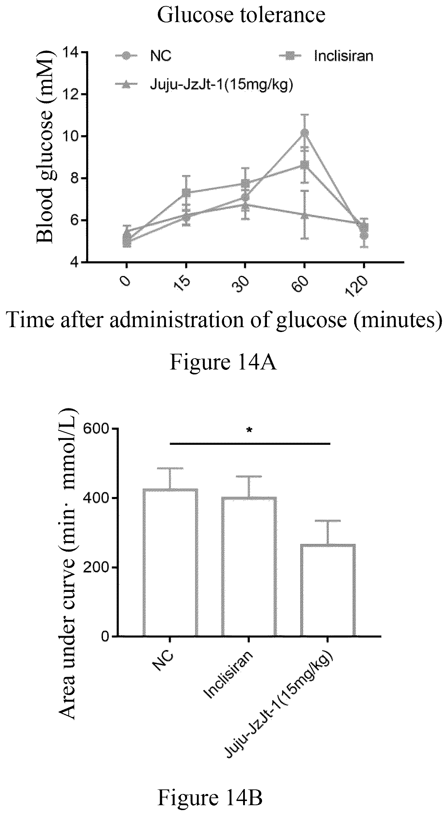

Figure 14A and Figure 14B show the effect of the small RNA Juju-JzJt-1 of the present invention on oral glucose tolerance in the hyperlipidemic cynomolgus monkey model compared with Inclisiran; NC represents the negative control group; Inclisiran represents the positive control group; Juju-JzJt-1 (15 mg/kg) represents the experimental group;

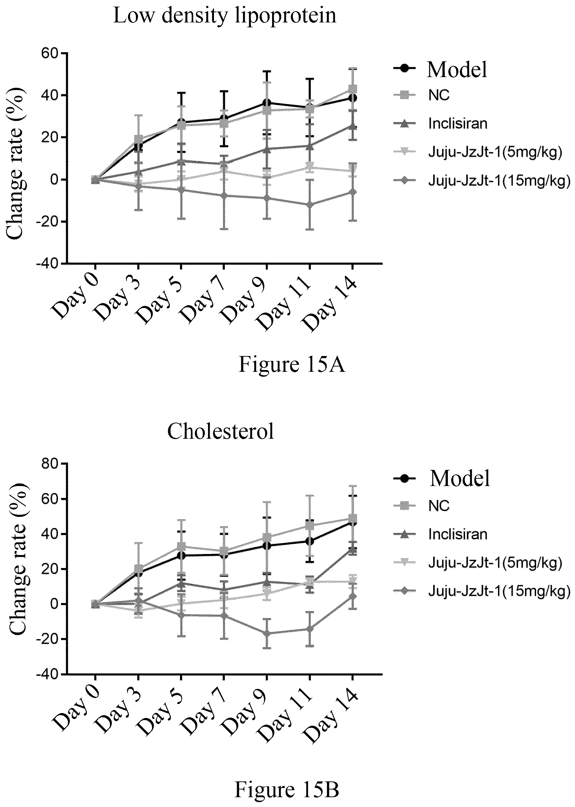

Figure 15A and Figure 15B show the effect of the small RNA Juju-JzJt-1 of the present invention on serum low density lipoprotein and cholesterol in the hyperlipidemic cynomolgus monkey model compared with Inclisiran; wherein Model represents the hyperlipidemia model group; NC represents the negative control group; Inclisiran represents the positive control group; Juju-JzJt-1 (5 mg/kg, 15 mg/kg) represents the experimental groups;

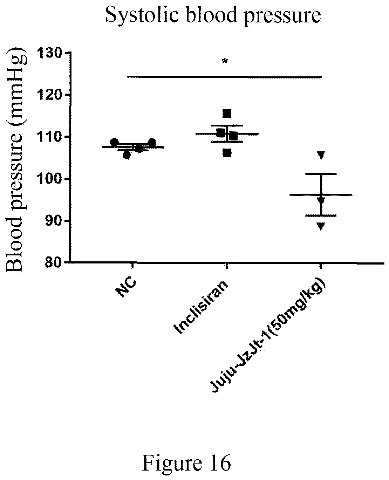

Figure 16 shows the effect of the small RNA Juju-JzJt-1 of the present invention on systolic blood pressure in the hyperlipidemic cynomolgus monkey model; wherein NC represents the negative control group; Inclisiran represents the positive control group; Juju-JzJt-1 (50 mg/kg) represents the experimental group;

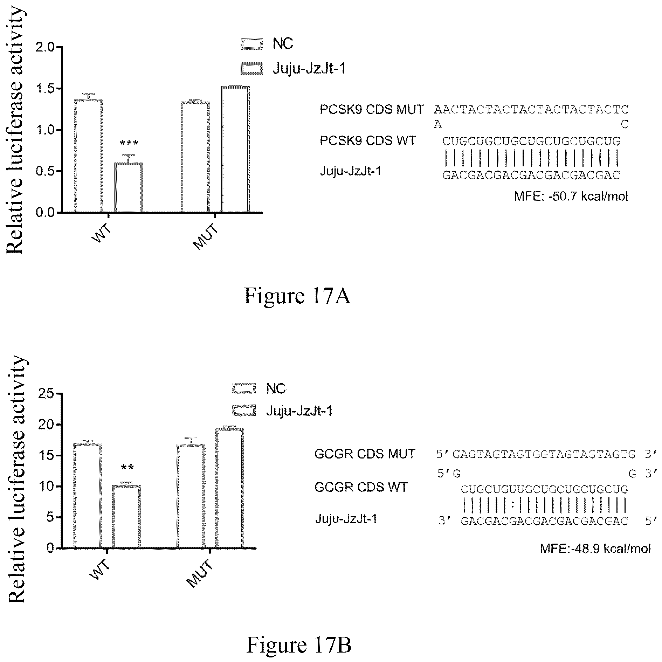

Figure 17A and Figure 17B show that the small RNA Juju-JzJt-1 of the present invention exerts a hypolipidemic effect by targeting proprotein convertase subtilisin/kexin type 9 (PCSK9) and exerts a hypoglycemic effect by glucagon receptor (GCGR), wherein WT represents PCSK9 CDS (protein-coding region) wild-type or GCGR CDS wild-type (Figure 17A and Figure 17B); MUT represents PCSK9 CDS mutant (or GCGR CDS mutant) (Figure 17A and Figure 17B); NC represents the negative control; Juju-JzJt-1 represents addition of the small RNA of the present invention.

### DETAILED DESCRIPTION OF THE INVENTION

The examples are incorporated below for further description of the present invention, but these examples do not limit the scope of the present invention. The experimental methods with unspecified conditions in the examples of the present invention generally follow conventional conditions, or according to the conditions recommended by the raw material or commodity manufacturer. The reagents without specified sources are conventional reagents purchased from the market.

### Example 1. Screening and clustering of PCSK9-targeting small RNAs derived from medicinal plants

The inventors studied the targeting effect of the small RNAs obtained by the present inventors from medicinal plants on proprotein convertase subtilisin/kexin type 9 (PCSK9) by a dual luciferase reporter detection system, so as to study its potential to exert a hypoglycemic effect.

293T (human embryonic kidney cells) cells were cultured, and transfected with the small RNAs to be screened and NC, respectively. The cells were then transfected with PCSK9 full-length dual luciferase reporter plasmid, and detected for dual luciferase activity after 48 hours.

These small RNAs could significantly reduce the dual luciferase activity by binding to PCSK9 (the NCBI number of said PCSK9 was NM_174936.4) compared with NC, demonstrating that these small RNAs may have a hypoglycemic function (see Figure 1A to Figure 1H for the results). Wherein, the specific sequences of the small RNAs were as shown in Table 1. PCSK9-sRNA-7 was selected as the research object for subsequent implementation, which was also named as Juju-JzJt-1 due to its origin.

Classification was done according to the different positions of the PCSK9 transcripts targeted by the small RNAs, and the classification results were as shown in Table 2. At the same time, the small RNAs SEQ ID NO: 1-SEQ ID NO: 7 of the present invention could exert an effect by targeting GCGR.

**Table 1. Sequences of PCSK9-targeting small RNAs derived from medicinal plants**

| Name | Sequence | SEQ ID NO: |
|---|---|---|
| PCSK9-sRNA-1 | AGCAGCAGCAGCAGCAGC | 1 |
| PCSK9-sRNA-2 | AGCAGCAGCAGCAGCAGCA | 2 |
| PCSK9-sRNA-3 | AGCAGCAGCAGCAGCAGCAG | 3 |
| PCSK9-sRNA-4 | CAGCAGCAGCAGCAGCAG | 4 |
| PCSK9-sRNA-5 | CAGCAGCAGCAGCAGCAGC | 5 |
| PCSK9-sRNA-6 | CAGCAGCAGCAGCAGCAGCA | 6 |
| PCSK9-sRNA-7 (Juju-JzJt-1) | CAGCAGCAGCAGCAGCAGCAG | 7 |
| PCSK9- sRNA-27 | UCGACGCGGGCUCUGCUCGUU | 8 |
| PCSK9- sRNA-28 | CCGUGCGCUGGAUUAUGACUG | 9 |
| PCSK9- sRNA-29 | UGGGCGGCAUUGUCUGCGGAU | 10 |
| PC SK9-sRNA-30 | UGCAGCCCCAAUCGGGCGGUA | 11 |
| PCSK9-sRNA-31 | UGCGGCUGGAUCACCUCCUUU | 12 |
| PCSK9-sRNA-32 | CGCGCUUGACUGCGAGACAGA | 13 |
| PCSK9-sRNA-33 | AUGGCUGUCGUCAGCUCGUGU | 14 |
| PCSK9-sRNA-36 | CCGGCGGACUGCUCGAGCUGC | 15 |
| PCSK9-sRNA-39 | CUGCCGGCGGACUGCUCGAGC | 16 |
| PCSK9-sRNA-40 | CCCAGCCCCGAACCCGUCGGC | 17 |
| PCSK9-sRNA-41 | CGGCUGUCGGUGGUCUGCUCG | 18 |
| PCSK9-sRNA-55 | GGUGGCUGUAGUUUAGUGGUA | 19 |
| PCSK9-sRNA-58 | AGCUUCAGUUGUCCGAUUUGU | 20 |
| PCSK9-sRNA-62 | AGGGCUGUAGCUCAGUUGGUA | 21 |
| PCSK9-sRNA-63 | CCUCGGCCGGAUGCGGAACGG | 22 |
| PCSK9-sRNA-68 | CCGACCCCGAUCUUCUGUGAA | 23 |
| PCSK9-sRNA-71 | GCUCGUAGUUGGACCUUGGGU | 24 |
| PCSK9-sRNA-72 | GCUCGUAGUUGGACCUUGGGA | 25 |
| PCSK9-sRNA-75 | CCCUGCGUCGCUCCGAUUCGU | 26 |
| PCSK9-sRNA-77 | UCUUCGGAAUUUGAAGCUAGA | 27 |
| PCSK9-sRNA-79 | UCCUCGAUGGUCUAACGGUCA | 28 |
| PCSK9-sRNA-80 | CGGCUGUCGGCGAACUGCUCG | 29 |
| PCSK9-sRNA-81 | ACACGGUCCAGACUCCUACGG | 30 |
| PCSK9-sRNA-82 | CGUUUCACGUCGGGUUCACCA | 31 |
| PCSK9-sRNA-83 | AAUGGCACCGUAACUUCGGGA | 32 |
| PCSK9-sRNA-84 | CGGAAGCCGGGUUACGGUGCC | 33 |
| PCSK9-sRNA-85 | CGCCGUCCGAAUUGUAGUCUG | 34 |
| PCSK9-sRNA-86 | UGGCUGUCGUCAGCUCGUGUU | 35 |
| PCSK9-sRNA-87 | CGGAAUCCGCUAAGGACUGUG | 36 |
| PCSK9-sRNA-88 | UGCGCCUCGGUCGGAUGUGGA | 37 |
| PCSK9-sRNA-90 | CUCCCCAUCCGGCCCGUCUUG | 38 |
| PCSK9-sRNA-91 | CCGGUGCGUUCCUGACAGUCU | 39 |
| PCSK9-sRNA-93 | UGCCUGGCGGCUGUAGCGCGG | 40 |
| PCSK9-sRNA-94 | CGAAUCCUGGCUGUUGUAUGU | 41 |
| PCSK9-sRNA-95 | CAUGCCUGUCCGAGCGUCAUU | 42 |
| PCSK9-sRNA-96 | CAUGCCUGUUCGAGCGUCAUU | 43 |
| PCSK9-sRNA-98 | AUCGGCUCGGGACAUUGACCG | 44 |
| PCSK9-sRNA-99 | UAUUCUGGUGUCCUAGGCGUA | 45 |
| PCSK9-sRNA-100 | CCUUCGGAGUUUGAAGCUAGA | 46 |
| PCSK9-sRNA-101 | CGGACUGCUCGAGCUGCUUCC | 47 |
| PCSK9-sRNA-102 | AUGCGAGAGUAGGGAACUGCC | 48 |
| PCSK9-sRNA-103 | CUUGACUCUAGUCCGACUUUG | 49 |
| PCSK9-sRNA-104 | AUCGACUCGGGGCGUGGACCG | 50 |
| PCSK9-sRNA-107 | GUGGCUGUAGUUUAGUGGUAA | 51 |
| PCSK9-sRNA-109 | GCUCGUAGUUGGACUUUGGGU | 52 |
| PCSK9-sRNA-110 | GCUCGUAGUUGGACUUUGGGA | 53 |
| PCSK9-sRNA-111 | UCCUCGAUGGUCUAACGGCUA | 54 |
| PCSK9-sRNA-112 | CGGGCUUUGCCCGGUGCUCUG | 55 |
| PCSK9-sRNA-113 | UGUGAGCUCGAUGAGUAGGGC | 56 |
| PCSK9-sRNA-114 | ACACGGCCCAGACUCCUACGG | 57 |
| PCSK9-sRNA-115 | GUGGAGCGGUGAAAUGCGUAG | 58 |
| PCSK9-sRNA-116 | CCCGUCGGCUGUCGGUGGACU | 59 |
| PCSK9-sRNA-117 | UAGGUAGCGCCUCAUGUAUCA | 60 |
| PCSK9-sRNA-118 | CGAUUCUCGGAUUGGACACCA | 61 |
| PCSK9-sRNA-119 | CUCACUGGUCGAGUCGGCCUG | 62 |
| PCSK9-sRNA-122 | AUUCCGGUCCUAUUCUGUUGG | 63 |
| PCSK9-sRNA-123 | GUGUUGACUUCAGUUUUAUGA | 64 |
| PCSK9-sRNA-124 | AGGACGUGCUAAUCUGCGAAA | 65 |
| PCSK9-sRNA-125 | AAAGUUGGGGGCUCGAAGACG | 66 |
| PCSK9-sRNA-126 | AUGCCUGUUUGAGCGUCAUUU | 67 |
| PCSK9-sRNA-127 | CGGCUGGAUCACCUCCUUUCU | 68 |
| PCSK9-sRNA-131 | CGGACAAGGGGAAUCCGACUG | 69 |
| PCSK9-sRNA-132 | CGCCUCGUGAACUCAUCUUCG | 70 |
| PCSK9-sRNA-135 | CCCCAUGCCGCACCGAUUCGU | 71 |
| PCSK9-sRNA-136 | UCCUGUACUGAGCUGCCCCGA | 72 |
| PCSK9-sRNA-137 | GCACCGGCUAACUCUGUGCCA | 73 |
| PCSK9-sRNA-276 | GCCUGGGCGGAGCGGUCGUCGGUGCA | 74 |
| PCSK9-sRNA-1595 | UGCAGCCCCAAUAGGGCGGUA | 75 |
| | CTGCTGCTGCTGCTGCTGCTG | 76 |
| | CTGCTGTTGCTGCTGCTGCTG | 77 |

Note: the sequences as shown in Table 1 are written in the 5' to 3' direction of the small RNAs.

**Table 2. Grouping of the small RNAs**

| Transcripts and positions being targeted | SEQ ID NO: |
|---|---|
| *PCSK9* 3'UTR | SEQ ID NOs: 11, 17, 20, 22, 32, 34, 38, 44, 50, 55, 62, 65, 71, 72, 75 |
| *PCSK9* 5'UTR | SEQ ID NOs: 8, 9, 16, 39, 40, 42, 43, 47, 67, 73 |
| *PCSK9* CDS, *GCGR* CDS | SEQ ID NOs: 1, 2, 3, 4, 5, 6, 7 |
| *PCSK9* CDS | SEQ ID NOs: 10, 12, 13, 14, 15, 18, 19, 21, 23, 24, 25, 26, 27, 28, 29, 30, 31, 33, 35, 36, 37, 41, 45, 46, 48, 49, 51, 52, 53, 54, 56, 57, 58, 59, 60, 61, 63, 64, 66, 68, 69, 70, 74 |

In the following examples, the inventors further validated the function of the small RNA Juju-JzJt-1. The small RNA used in cell experiments was a double-stranded RNA synthesized by complementation of the provided sequence, and the small RNA used in all animal experiments was a single-stranded small RNA.

### Example 2. Reverse transcription-real-time PCR and western blot experiments after transfection of HepG2 with small RNA

HepG2 cells were cultured in 24-well plates/12-well plates until reaching 70-80% confluency, and transfected with NC, double-stranded demodified Inclisiran, or the small RNAs PCSK9-3, PCSK9-5, PCSK9-6, and the small RNA Juju-JzJt-1 in gradient doses. The total RNA or protein of the cells was collected after 24 hours or 48 hours of culture. Relative quantification of *PCSK9* and *LDLR* messenger RNAs was performed by reverse transcription-real-time PCR (see Figure 2A and Figure 2B) and relative quantification of PCSK9 and LDLR proteins was performed by western blot, and statistical analysis was performed (see Figure 3A, Figure 3B and Figure 3C).

### Example 3. Uptake experiment of human-derived low density lipoprotein labeled with red fluorescence in HepG2

HepG2 cells were cultured in 12-well plates until reaching 50-60% confluency and transfected with NC, double-stranded demodified Inclisiran, or the small RNA Juju-JzJt-1. Human-derived low density lipoprotein labeled with red fluorescence was added after 48 hours of culture, and the cells were cultured for an additional 6 hours. The cells were imaged under a fluorescence microscope and the percentage of positive cells was calculated (see Figure 4A and Figure 4B).

### Example 4. Preparation of herbal medicine sphingosine(d18:1) - Juju-JzJt-1

300 µL of nuclease-free water was added to 5 nmol of dry powder of the small RNA Juju-JzJt-1 (SEQ ID NO: 1), and then 10 µL lipid solution of 10 mg/mL sphingosine(d18:1) (D-erythro-sphingosine, purchased from Avanti, Cat. No. 860490P) was added. The mixture was shaken and mixed well, heated in a water bath at 90°C for 15 minutes, and cooled to room temperature, so as to prepare a mixture comprising the small RNA Juju-JzJt-1 and sphingosine(d18:1).

A negative control mixture was prepared in a similar manner, in which the small RNA Juju-JzJt-1 was replaced by the irrelative control sequence NC, and the rest of the steps were the same as above.

### Example 5. Experiment of glucose tolerance in high-fat model ob/ob mice improved by herbal medicine sphingosine(d18:1) - Juju-JzJt-1 of the present invention compared with Inclisiran

ob/ob model group: no treatment was given;
NC group: herbal medicine sphingosine(d18:1) - NC was administered by gavage at a dose of 2 mg/kg for 14 consecutive days;
Inclisiran group: Inclisiran was injected subcutaneously at a dose of 15 mg/kg for once on the first day of the experiment;
Juju-JzJt-1 group: herbal medicine sphingosine(d18:1) - Juju-JzJt-1 was administered by gavage at a dose of 0.2 mg/kg, 0.6 mg/kg or 2 mg/kg for 14 consecutive days;
PCSK9-3 group: herbal medicine sphingosine (d18:1) - PCSK9-3 was administered by gavage at a dose of 2 mg/kg for 14 consecutive days.

### Oral glucose tolerance test

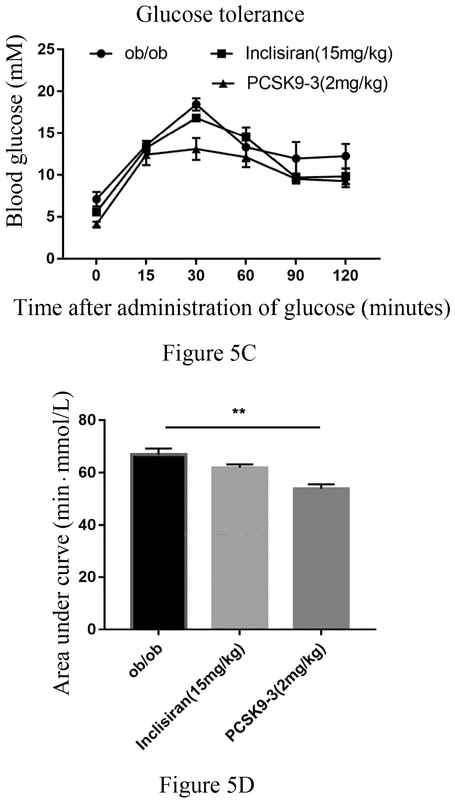

Mice were fasted for 16 hours and then weighed. The amount of oral glucose was calculated according to body weight (standard for the amount to be injected: 1200 mg/kg, concentration of glucose solution: 120 mg/mL). The fasting blood glucose at this time point was first measured and labeled as blood glucose at 0 minute. The mouse was restrained and the needle was punctured into the abdominal cavity at an angle of 45° to the abdomen. The glucose solution was slowly injected and timing was started. The blood glucose level (in the blood taken from the mouse tail) was measured at 15 minutes, 30 minutes, 45 minutes, 60 minutes, 90 minutes and 120 minutes, respectively, and a line chart was plotted (see Figure 5A and Figure 5C).

In the oral glucose tolerance test of high-fat model ob/ob mice, there was no change in glucose tolerance in mice treated with 15 mg/kg Inclisiran, while treatment with 2 mg/kg Juju-JzJt-1 and PCSK9-3 significantly inhibited the rise of blood glucose, kept blood glucose in a low and stable state, and significantly enhanced the ability of glucose metabolism, compared with the model group (see Figure 5B and Figure 5D). In summary, the small RNA Juju-JzJt-1 of the present invention not only had a good effect on lowering blood lipids, but also improved glucose tolerance in mice, while Inclisiran had no obvious effect on improving glucose tolerance.

### Example 6. Experiment of insulin tolerance in high-fat model ob/ob mice improved by herbal medicine sphingosine(d18:1) - Juju-JzJt-1 of the present invention compared with Inclisiran

Mice were fasted for 4 hours and then weighed. The amount of insulin to be injected intraperitoneally was calculated according to body weight (standard for the amount to be injected: 1.2 IU/kg, concentration of insulin: 0.12 IU/mL). The fasting blood glucose at this time point was first measured and labeled as blood glucose at 0 minute. The mouse was restrained and the needle was punctured into the abdominal cavity at an angle of 45° to the abdomen. Insulin was slowly injected and timing was started. The blood glucose level (in the blood taken from the mouse tail) was measured at 15 minutes, 30 minutes, 60 minutes and 90 minutes, respectively, and a line chart was plotted (see Figure 6A and Figure 6B).

In the insulin tolerance test of high-fat model ob/ob mice, there was no change in glucose levels in mice treated with 15 mg/kg Inclisiran, while 2 mg/kg Juju-JzJt-1 significantly enhanced insulin sensitivity, compared with the model group. In summary, the small RNA Juju-JzJt-1 of the present invention had a significant enhancing effect on the body's sensitivity to insulin.

### Example 7. Experiment of serum triglycerides, low density lipoprotein and cholesterol in high-fat model ob/ob mice improved by herbal medicine sphingosine(d18:1) - Juju-JzJt-1 of the present invention compared with Inclisiran

After 14 days of dosing, mice were subjected to retro-orbital blood collection and serum was separated. The triglycerides, low density lipoprotein and cholesterol in the serum were detected by kits for comparison of the contents between different dosing groups, and dot plots were plotted (see Figure 7A, Figure 7B and Figure 7C).

Among the serum contents of the three indicators detected in high-fat model ob/ob mice, serum triglyceride levels of mice were significantly reduced by both 15 mg/kg Inclisiran and Juju-JzJt-1 treatment compared with the negative control group, and 2 mg/kg Juju-JzJt-1 was superior than Inclisiran in terms of lowering low density lipoprotein and cholesterol levels.

### Example 8. Effect of herbal medicine sphingosine(d18:1) - Juju-JzJt-1 of the present invention on the expression levels of PCSK9 and LDLR in the liver of high-fat model ob/ob mice

After 14 days of dosing, total tissue RNA or protein was collected from mice's livers. Relative quantification of *PCSK9* and *HepG2* messenger RNAs was performed by reverse transcription-real-time PCR (see Figure 8A and Figure 8B) and relative quantification of PCSK9 and HepG2 proteins was performed by western blot, and statistical analysis was performed (see Figure 9A, Figure 9B and Figure 9C).

### Example 9. Ameliorating effect of herbal medicine sphingosine(d18:1) - Juju-JzJt-1 of the present invention on liver lesions in high-fat model ob/ob mice

After 14 days of dosing, mice's livers were fixed in 4% paraformaldehyde solution, and then stained with oil red O.

The results of liver oil red O staining showed that (see Figure 10) the liver sections of mice in high-fat model group and negative control group had obvious lipid accumulation, and the treatment of both 15 mg/kg Inclisiran and 2 mg/kg small RNA Juju-JzJt-1 significantly ameliorated this lipid accumulation in liver.

### Example 10. Ameliorating effect of herbal medicine sphingosine(d18:1) - Juju-JzJt-1 of the present invention on liver inflammatory factors in high-fat model ob/ob mice

After 14 days of dosing, mice's livers were placed in strong RIPA lysis buffer and ground with a benchtop tissue homogenizer. The supernatant was separated and detected by enzyme-linked immunosorbent assay kits for the inflammatory factors interleukin-1β, interleukin-6 and tumor necrosis factor-α in the liver. The contents of the three inflammatory factors in different groups were compared, and dot plots were plotted (see Figure 11A, Figure 11B and Figure 11C).

Among the liver tissue contents of the inflammatory factors detected in high-fat model ob/ob mice, both 15 mg/kg Inclisiran and 2 mg/kg Juju-JzJt-1 significantly reduced the above three inflammatory factors compared with the model group. In summary, the small RNA Juju-JzJt-1 of the present invention could significantly reduce the level of inflammatory factors in liver tissue and improve liver function in ob/ob mice.

### Example 11. Experiment of glucose tolerance improved by herbal medicine sphingosine (d18:1) - Juju-JzJt-1 of the present invention in beagle dogs

15 beagle dogs (provided by Beijing Fangyuanyuan Farm) were randomly divided into the following 3 groups:
Blank group: no treatment was given;
NC group: negative control group, herbal medicine sphingosine(d18:1) - NC was administered by gavage at a dose of 5 mg/kg for 7 consecutive days;
Juju-JzJt-1 group: herbal medicine sphingosine(d18:1) - Juju-JzJt-1 was administered by gavage at a dose of 5 mg/kg for 7 consecutive days;

### Oral glucose tolerance test

Beagle dogs were fasted for 8 hours and weighed. After anesthesia by intramuscular injection of anesthetic, fasting blood glucose was first measured and labeled as blood glucose at 0 minute. 8 ml/kg of 50% glucose solution was administered by gavage according to body weight, and intravenous blood was collected at 15 minutes, 30 minutes, 60 minutes, 90 minutes, 120 minutes and 180 minutes, respectively. The blood glucose of animals was immediately detected by a glucose meter, and a line chart was plotted (see Figure 12A and Figure 12B).

In the oral glucose tolerance test in beagle dogs, the treatment of 5 mg/kg sphingosine(d18:1) - Juju-JzJt-1 significantly inhibited the rise of blood glucose, kept blood glucose in a low and stable state, and significantly enhanced the ability of glucose metabolism, compared with the negative control group. In summary, the small RNA Juju-JzJt-1 of the present invention not only had a good effect on lowering blood lipids, but also improved glucose tolerance in mice, while Inclisiran had no obvious effect on improving glucose tolerance.

### Example 12. Experiment of serum low density lipoprotein, cholesterol and high-density lipoprotein improved by herbal medicine sphingosine(d18:1) - Juju-JzJt-1 of the present invention in beagle dogs

Before dosing and 7 days after dosing, whole blood was collected and serum was separated. The triglycerides, low density lipoprotein and cholesterol in the serum were detected by an automatic biochemical analyzer. The change rate of indicators in different dosing groups after dosing was calculated with the values before dosing as baseline, and line plots were plotted (see Figure 13A, Figure 13B and Figure 13C).

5 mg/kg Juju-JzJt-1 could significantly reduce the serum low density lipoprotein and cholesterol levels and increase the serum high density lipoprotein level in beagle dogs, compared with the model group or negative control group.

### Example 13. Experiment of glucose tolerance improved by herbal medicine sphingosine(d18:1) - Juju-JzJt-1 of the present invention in hyperlipidemic cynomolgus monkeys

24 male hyperlipidemic cynomolgus monkeys (provided by Jiangsu Kemaqi) were fed high-fat diet during the experiment and were randomly divided into the following 5 groups:
Model group: no treatment was given;
NC group: negative control group, herbal medicine sphingosine(d18:1) - NC-small RNA was administered by gavage at a dose of 5 mg/kg for 14 consecutive days;
Inclisiran group: positive control group, Inclisiran was injected subcutaneously at a dose of 5 mg/kg for once on the first day of the experiment;
Juju-JzJt-1 group: herbal medicine sphingosine(d18:1) - Juju-JzJt-1 was administered by gavage at a dose of 5 mg/kg, 15 mg/kg or 50 mg/kg for 14 consecutive days;

### Oral glucose tolerance test

Cynomolgus monkeys were fasted for 12 hours and weighed. The fasting blood glucose was measured and labeled as the blood glucose at 0 minute. 10 ml/kg of 30% glucose solution was administered by gavage according to body weight. Blood was collected from the tail vein at 5 minutes, 15 minutes, 30 minutes, 60 minutes and 120 minutes, respectively. The blood glucose of animals were immediately detected by a glucose meter and a line chart was plotted (see Figure 14A and Figure 14B).

In the oral glucose tolerance test in cynomolgus monkeys, treatment with 15 mg/kg sphingosine (d18:1) - Juju-JzJt-1 significantly inhibited the rise of blood glucose and kept blood glucose in a stable state compared with the negative control group, while Inclisiran had no effect on improving blood glucose.

### Example 14. Experiment of serum low density lipoprotein and cholesterol improved by herbal medicine sphingosine(d18:1) - Juju-JzJt-1 of the present invention in hyperlipidemic cynomolgus monkeys

Before dosing and during dosing, whole blood was collected and serum was separated. The low density lipoprotein and cholesterol in the serum were detected by an automatic biochemical analyzer. The change rate of indicators in different dosing groups after dosing was calculated with the values before dosing as baseline, and line plots were plotted (see Figure 15A and Figure 15B).

Both 5 mg/kg and 15 mg/kg Juju-JzJt-1 significantly reduced the serum levels of low density lipoprotein and cholesterol in cynomolgus monkeys compared with the model group or the negative control group. The effect of 5 mg/kg Juju-JzJt-1 was comparable to that of Inclisiran, and the effect of 15 mg/kg Juju-JzJt-1 was superior. In summary, the small RNA Juju-JzJt-1 of the present invention could play a role in improving blood lipids in primates, and the effect was not inferior to Inclisiran.

### Example 15. Experiments of systolic blood pressure improved by herbal medicine sphingosine(d18:1) - Juju-JzJt-1 of the present invention in hyperlipidemic cynomolgus monkeys

Systolic blood pressure in cynomolgus monkeys was measured before dosing on Day 11 of the experiment, and a dot plot was plotted (see Figure 16).

50 mg/kg Juju-JzJt-1 reduced the systolic blood pressure level in cynomolgus monkeys compared with the negative control group, while Inclisiran had no effect on improving blood pressure. In summary, the small RNA Juju-JzJt-1 of the present invention could play a role in improving blood lipids, blood sugar and blood pressure in primates, and the effect was not inferior to Inclisiran.

### Example 16. Hypolipidemic effect by targeting PCSK9 and hypoglycemic effect by targeting GCGR of the small RNA Juju-JzJt-1

293T cells were cultured and transfected with the small RNAs Juju-JzJt-1 and NC, and with PCSK9 or GCGR dual luciferase reporter plasmids, respectively. The cells were detected for dual luciferase activity after 48 hours (see Figure 17A and Figure 17B).

Juju-JzJt-1 significantly reduced dual luciferase activity by binding complementarily with the protein-coding region sequence CTGCTGCTGCTGCTGCTGCTG (SEQ ID NO: 76) of PCSK9 (the NCBI No. of said PCSK9 was NM_174936.4) compared with NC (See Figure 17A). However, base mutation of the binding region between Juju-JzJt-1 and PCSK9 eliminated the binding ability, demonstrating that the small RNA Juju-JzJt-1 exerted a hypolipidemic effect by targeting PCSK9.

Juju-JzJt-1 significantly reduced dual luciferase activity by binding complementarily with the protein-coding region sequence CTGCTGTTGCTGCTGCTGCTG (SEQ ID NO: 77) of GCGR (the NCBI No. of said GCGR was NM_000160.5) compared with NC (See Figure 17B). However, base mutation of the binding region between Juju-JzJt-1 and GCGR eliminated the binding ability, demonstrating that the small RNA Juju-JzJt-1 exerted a hypoglycemic effect by targeting GCGR.

## Claims

1. An isolated nucleic acid molecule comprising or consisting of the following sequence:
A) a nucleotide sequence as shown in any one of SEQ ID NO: 1 to SEQ ID NO: 75;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 75;
C) a nucleotide sequence having up to 10 nucleotide substitutions, deletions, or additions compared with a nucleotide sequence as shown in any one of SEQ ID NO: 1 to SEQ ID NO: 75, preferably a nucleotide sequence having 9, 8, 7, 6, 5, 4, 3, 2 or 1 nucleotide substitution, deletion, or addition compared with a nucleotide sequence as shown in any one of SEQ ID NO: 1 to SEQ ID NO: 75,
D) a nucleotide sequence capable of hybridizing to a nucleotide sequence as shown in any one of SEQ ID NO: 1 to SEQ ID NO: 75 under stringent conditions, or
F) a nucleotide sequence targeting the PCSK9 or GCGR gene, preferably a nucleotide sequence capable of binding complementarily to a nucleotide sequence as shown in SEQ ID NO: 76 or 77.

2. The isolated nucleic acid molecule according to claim 1, which exerts a hypolipidemic effect by targeting PCSK9, preferably by binding complementarily to the CDR, 3' UTR or 5' UTR of PCSK9 to reduce the expression of PCSK9, and preferably by binding complementarily to the CDS region of PCSK9 as shown in SEQ ID NO: 76 to reduce the expression of PCSK9.

3. The isolated nucleic acid molecule according to any one of claim 1 or 2, which is an RNA molecule or a DNA molecule.

4. The isolated nucleic acid molecule according to claim 3, which is a small RNA molecule, preferably a small RNA molecule with a length of 18-24 nucleotides, preferably a small RNA molecule with a length of 19, 20, 21, 22, 23, or 24 nucleotides.

5. An isolated small RNA molecule targeting the PCSK9 gene and comprising or consisting of the following sequence:
A) a nucleotide sequence as shown in any one of SEQ ID NO: 1 to SEQ ID NO: 75;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 75;
C) a sequence having up to 10 nucleotide substitutions, deletions, or additions compared with a nucleotide sequence as shown in any one of SEQ ID NO: 1 to SEQ ID NO: 75, preferably a sequence having 9, 8, 7, 6, 5, 4, 3, 2 or 1 nucleotide substitution, deletion, or addition,
D) a sequence capable of hybridizing to a nucleotide sequence as shown in any one of SEQ ID NO: 1 to SEQ ID NO: 75 under stringent conditions, or
E) a nucleotide sequence capable of binding complementarily to a nucleotide sequence as shown in SEQ ID NO: 76 or 77.

6. An isolated small RNA molecule targeting a nucleotide sequence as shown in SEQ ID NO: 76 or 77, preferably, the isolated small RNA molecule comprises or consists of the following sequence:
A) a nucleotide sequence as shown in any one of SEQ ID NO: 1 to SEQ ID NO: 7;
B) a sequence having at least 70%, at least 75%, at least 80%, at least 85%, at least 90%, at least 95%, 96%, 97%, 98%, 99% sequence identity with a nucleotide sequence as shown in any one of SEQ ID NO: 1 to SEQ ID NO: 7;
C) a sequence having up to 10 nucleotide substitutions, deletions, or additions compared with a nucleotide sequence as shown in any one of SEQ ID NO: 1 to SEQ ID NO: 7, preferably a sequence having 9, 8, 7, 6, 5, 4, 3, 2 or 1 nucleotide substitution, deletion, or addition; or
D) a sequence capable of hybridizing to a nucleotide sequence as shown in any one of SEQ ID NO: 1 to SEQ ID NO: 7 under stringent conditions.

7. The isolated small RNA molecule according to claim 6, which exerts a hypolipidemic, hypoglycemic and/or hypotensive function by targeting PCSK9 CDS and/or GCGR CDS.

8. The isolated small RNA molecule according to any one of claims 5-7, which is a single-stranded or a double-stranded small RNA molecule.

9. A precursor miRNA, which may be processed within the host into the isolated small RNA molecule according to any one of claims 5-8.

10. A polynucleotide, which may be transcribed by the host to form the precursor miRNA according to claim 9.

11. An expression vector comprising the isolated nucleic acid molecule according to any one of claims 1-4, the isolated small RNA molecule according to any one of claims 5-8, the precursor miRNA according to claim 9, and/or the polynucleotide according to claim 10.

12. A host cell transfected with the expression vector according to claim 11.

13. A pharmaceutical composition comprising a therapeutic effective amount of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, or the host cell according to claim 12, as well as one or more pharmaceutically acceptable adjuvants, excipients and/or stabilizers, preferably, the pharmaceutical composition is used for administration via oral, intramuscular, intravenous, subcutaneous, percutaneous, intraarterial, intraperitoneal, intrapulmonary, intracerebrospinal, intraarticular, intrasynovial, intrathecal, intraventrical, and/or inhalation routes.

14. The pharmaceutical composition according to claim 13, wherein the content of the isolated nucleic acid molecule, the isolated small RNA molecule, the precursor miRNA, the polynucleotide, the expression vector or the host cell in the pharmaceutical composition is 0.1-1000 µM, preferably 3.0 µM-300 µM.

15. Use of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14 in the preparation of a medicament for treating a disease, said disease is selected from the group consisting of hyperlipidemia, hypertension, and diabetes.

16. Use of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14 in the preparation of a medicament for treating and/or preventing a disease, said disease is selected from diseases associated with elevated blood lipids, preferably, said disease is selected from the group consisting of atherosclerosis, coronary heart disease, myocardial infarction, cerebral hemorrhage, and cerebral thrombosis.

17. A method for treating a disease in a subject in need, including administering to the subject a therapeutically effective amount of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14, said disease is selected from the group consisting of hyperlipidemia, hypertension, and diabetes.

18. A method for treating a disease associated with abnormal gene expression, including administering to a subject a therapeutically effective amount of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14.

19. A method for inhibiting the expression and/or activity of a gene, including administering to a subject a therapeutically effective amount of the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14.

20. A method for treating and/or preventing a disease associated with elevated blood lipids in a subject in need, including administering to the subject the isolated nucleic acid molecule according to any one of claims 1-4, the isolated small RNA molecule according to any one of claims 5-8, the precursor miRNA according to claim 9, the polynucleotide according to claim 10, the expression vector according to claim 11, the host cell according to claim 12, or the pharmaceutical composition according to claim 13 or 14, said disease associated with elevated blood lipids is selected from the group consisting of atherosclerosis, coronary heart disease, myocardial infarction, cerebral hemorrhage, and cerebral thrombosis.
